# EUROPÄISCHE PATENTSCHRIFT

(11) **EP 0 612 724 B1**
(45) Veröffentlichungstag und Bekanntmachung des Hinweises auf die Patenterteilung: **27.12.1996**
(21) Anmeldenummer: 94102321.0
(22) Anmeldetag: 16.02.1994
(51) Int. Cl.: C07C 311/58, A61K 31/64

(54) **Substituierte Benzolsulfonylharnstoffe und -thioharnstoffe- Verfahren zu ihrer Herstellung und ihre Verwendung als Pharmazeutika**
Substituted benzenesulfonylureas and -thioureas, process for their preparation and their use as pharmaceuticals
Benzène sulfonylurées et -thiourées substituées, procédé pour leur préparation et leur utilisation comme produits pharmaceutiques

(30) Priorität: 23.02.1993 DE 4305450
(43) Veröffentlichungstag der Anmeldung: 31.08.1994
(73) Patentinhaber: HOECHST AKTIENGESELLSCHAFT, 65929 Frankfurt am Main (DE)
(72) Erfinder: Englert, Heinrich, Dr., D-65719 Hofheim/Taunus (DE); Mania, Dieter, Dr., D-61462 Königstein/Taunus (DE); Hartung, Jens, Dr., D-63110 Rodgau (DE); Gögelein, Heinz, Dr., D-60259 Frankfurt (DE); Kaiser, Joachim, Dr., D-60431 Frankfurt (DE); Linz, Wolfgang, Dr., D-55129 Mainz (DE); Wettlaufer, David, Dr., Phillipsburg, N.J. 08865 (US)

(56) Entgegenhaltungen:
- DE-A- 1 518 874
- DE-A- 2 413 514

## Beschreibung

Die Erfindung betrifft substituierte Benzolsulfonylharnstoffe und -thioharnstoffe I 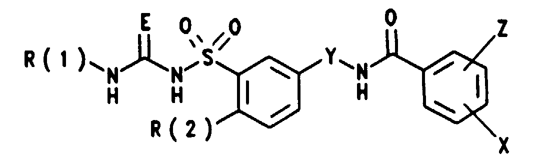 worin
- R(1): für Wasserstoff, Methyl, CH₂F, CHF₂ oder Trifluormethyl steht,
- R(2): Wasserstoff, F, Cl, Br, 1, (C₁-C₆)-Alkyl, (C₁-C₆)-Alkoxy, (C₁-C₆)-Mercaptoalkyl, (C₁-C₆)-Fluoralkoxy oder (C₁-C₆)-Fluoralkyl ist,
- E: für Sauerstoff oder Schwefel steht,
- Y: eine Kohlenwasserstoffkette der Formel:

-[CR(3)₂]ₙ-

mit R(3) = H oder (C₁-C₂)-Alkyl und n = 1, 2, 3 oder 4 ist,
- X: steht für Wasserstoff, F, Cl, Br, J oder (C₁-C₆)-Alkyl,
- Z: steht für F, Cl, Br, l, NO₂, (C₁-C₄)-Alkoxy oder (C₁-C₄)-Alkyl.

Der Begriff Alkyl beschreibt, sofern nicht anders angegeben, geradkettige, verzweigte oder cyclische gesättigte Kohlenwasserstoffreste mit einem bis sechs Kohlenstoffatomen. Der Cycloalkylrest kann zusätzlich Alkyl oder Trifluormethylsubstituenten tragen. Der Terminus Alkoxy steht für einen Ethersubstituenten mit einem geradkettigen, verzweigten oder cyclischen gesättigten Kohlenwasserstoffrest aus einem bis sechs Kohlenstoffatomen. Fluoralkyl beschreibt ein geradkettiges, verzweigtes oder cyclisches gesättigtes Kohlenstoffgerüst von einem bis sechs Kohlenstoffatomen in dem mindestens ein Wasserstoffatom des oben definierten Alkylrestes gegen Fluor ausgetauscht ist, maximal aber Perfluorsubstitution erreicht ist. Unter Fluoralkoxy wird ein Ethersubstituent verstanden, der einen Fluoralkylrest gemäß obiger Definition trägt. Als Halogensubstituent sind die Elemente Fluor, Chlor, Brom und Iod einsetzbar. Weiterhin können Verbindungen mit Chiralitätszentren in der Alkylseitenkette Y auftreten. In diesem Fall gehören sowohl die einzelnen Antipoden für sich, als auch eine Mischung der Enantiomere oder Diastereomere in unterschiedlichen Verhältnissen, sowie die dazugehörigen Mesoverbindungen oder Mischungen aus Mesoverbindungen, den Enantiomeren oder Diastereomeren zur Erfindung.

Ähnliche Sulfonylharnstoffe sind aus der deutschen Offenlegungsschrift 2 413 514 und der deutschen Patentschrift 1 518 874 bekannt. Die DE-OS 2 413 514 beschreibt ausschließlich Blutzucker-konditionierende Stoffe mit p-Substitution in der zentralen Phenylgruppe. Hinweise auf m-Substitution und einen Aminosubstituenten finden sich nicht.

Die DE-PS 1 518 874 beschreibt Blutzucker-senkende Sulfonylharnstoffe der Formel 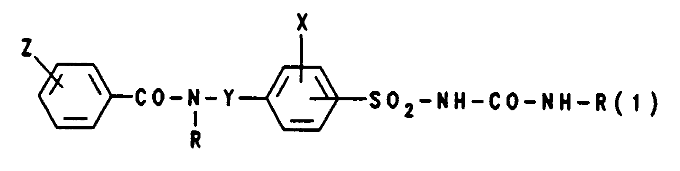 in denen die zentrale Phenylgruppe zwar auch m-substituiert und tri-substituiert sein kann, in denen jedoch R(1) nur (C₂-C₈)-Alkyl (neben vielen anderen Bedeutungen), in denen aber R(1) nie ein C₁-Substituent oder Wasserstoff sein kann.

In beiden Patent-Veröffentlichungen wird deren blutzuckersenkende Wirkung beschrieben. Als Prototyp solcher blutzuckersenkenden Sulfonylharnstoffe gilt das Glibenclamid, das als Mittel zur Behandlung des Diabetes mellitus therapeutisch verwendet wird und in der Wissenschaft ein vielbeachtetes Werkzeug zur Erforschung sogenannter ATP sensitiver Kaliumkanäle dient. Neben seiner blutzuckersenkenden Wirkung besitzt das Glibenclamid noch andere Wirkungen, die bislang therapeutisch noch nicht eingesetzt werden können die aber allesamt auf Blockade eben dieser ATP-sensitiven KaliumKanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann.

Aufgabe der vorliegenden Erfindung war es daher, Verbindungen zu synthetisieren, die eine gleich gute Herzwirkung wie Glibenclamid aufweisen, aber den Blutzucker in herzwirksamen Dosen oder Konzentrationen nicht oder deutlich geringer beeinflussen als Glibenclamid.

Als Versuchstiere zum Nachweis solcher Wirkungen eignen sich zum Beispiel Mäuse, Ratten, Meerschweinchen, Kaninchen, Hunde, Affen oder Schweine.

Die Verbindungen I dienen als Arzneimittelwirkstoffe in der Human- und Veterinärmedizin. Ferner können sie als Zwischenprodukte zur Herstellung weiterer Arzneimittelwirkstoffe verwendet werden.

Bevorzugt sind die Verbindungen I in denen sind
- R(1): Wasserstoff, Methyl, Trifluormethyl,
- R(2): Wasserstoff, (C₁-C₆)-Alkyl, (C₁-C₆)-Alkoxy, (C₁-C₆)-Mercaptoalkyl, (C₁-C₆)-Fluoralkyl, (C₁-C₆)-Fluoralkoxy oder F, Cl, Br, I,
- E: Sauerstoff oder Schwefel,
- Y: eine Kohlenwasserstoffkette der Formel -[CR(3)₂]ₙ- mit R(3) = H oder (C₁-C₂)-Alkyl und n = 1, 2, 3 oder 4,
- X: Wasserstoff, F, Cl oder (C₁-C₄)-Alkyl,
- Z: Nitro, F, Cl, (C₁-C₄)-Alkyl oder (C₁-C₄)-Alkoxy.

Besonders sind die Verbindungen I bevorzugt, in denen bedeuten:
- R(1): Wasserstoff, Methyl,
- R(2): (C₁-C₆)-Alkyl oder (C₁-C₆)-Alkoxy,
- E: Sauerstoff oder Schwefel,
- Y eine Kohlenwasserstoffkette der Formel: -[CR(3)₂]ₙ- mit R(3) = H oder (C₁-C₂)-Alkyl und n = 1, 2, 3 oder 4,
- X: Wasserstoff, F, Cl oder (C₁-C₄)-Alkyl,
- Z: Chlor und Fluor, (C₁-C₄)-Alkyl, (C₁-C₄)-Alkoxy.

Ganz besonders bevorzugt sind Verbindungen I, in denen bedeuten:
- R(1): Wasserstoff, Methyl, und
- R(2): Wasserstoff, Fluor oder Chlor,
- E: Sauerstoff oder Schwefel,
- Y: eine Kohlenwasserstoffkette der Formel: -[CR(3)₂]ₙ- mit R(3) = H oder (C₁-C₂)-Alkyl und n = 1, 2, 3 oder 4,
- X: Wasserstoff, F, Cl, (C₁-C₄)-Alkyl,
- Z: Chlor, Fluor, (C₁-C₄)-Alkyl, (C₁-C₄)-Alkoxy.

Ebenfalls besonders bevorzugt sind Verbindungen I, in denen bedeuten
- R(1): Wasserstoff, Methyl,
- R(2): (C₁-C₆)-Fluoralkyl, (C₁-C₆)-Fluoralkoxy, (C₁-C₆)-Mercaptoalkyl,
- E: Sauerstoff, Schwefel,
- Y: eine Kohlenwasserstoffkette der Formel: -[CR(3)₂]ₙ- mit R(3) = H oder (C₁-C₂)-Alkyl und n = 1, 2, 3 oder 4,
- X: Wasserstoff, F, Cl, (C₁-C₄)-Alkyl,
- Z: Chlor, Fluor, (C₁-C₄)-Alkyl, (C₁-C₄)-Alkoxy.

Insbesondere sind die Verbindungen I bevorzugt, in denen bedeuten:
- R(1): Wasserstoff, Methyl,
- R(2): Methoxy, Methyl,
- E: Sauerstoff oder Schwefel,
- Y: eine Kohlenwasserstoffkette der Formel -[CR(3)₂]ₙ- mit R(3) = H oder Methyl und n = 2 oder 3,
- X: Wasserstoff, F, Cl, (C₁-C₃)-Alkyl,
- Z: Chlor oder Fluor, (C₁-C₃)-Alkyl, (C₁-C₃)-Alkoxy.

Die Verbindungen I der vorliegenden Erfindung sind wertvolle Arzneimittel zur Behandlung von Herzrhythmusstörungen unterschiedlichster Genese und zur Verhinderung des arrhythmisch bedingten, plötzlichen Herztodes und können daher als Antiarrhythmika Verwendung finden. Beispiele arrhythmischer Störungen des Herzens sind supraventrikuläre Rhythmusstörungen, wie etwa Vorhof-Tachykardien, Vorhofflattern oder paroxysmale supraventrikuläre Rhythmusstörungen, oder ventrikuläre Rhythmusstörungen, wie ventrikuläre Extrasystolen, insbesondere aber lebensbedrohende ventrikuläre Tachykardien oder das besonders gefährliche Kammerflimmern. Sie eignen sich insbesondere für solche Fälle, in denen Arrhythmien Folge einer Verengung eines Koronargefäßes sind, wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztodes geeignet. Weitere Krankheitsbilder, bei denen derartige Rhythmusstörungen und/oder der plötzliche, arrhythmisch bedingte Herztod eine Rolle spielen sind beispielsweise die Herzinsuffizienz oder die Herzhypertrophie als Folge eines chronisch erhöhten Blutdruckes.

Darüberhinaus können die Verbindungen I eine verminderte Kontraktilität des Herzens positiv beeinflussen. Hierbei kann es sich um ein krankheitsbedingtes Nachlassen der Herzkontraktilität handeln, beispielsweise bei Herzinsuffizienz, aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch kardioplegische Lösungen erforderlich machen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die Erfindung betrifft weiterhin ein Verfahren zur Herstellung der Verbindungen I dadurch gekennzeichnet, daß man
(a) aromatische Sulfonamide der Formel II oder deren Salze der Formel III 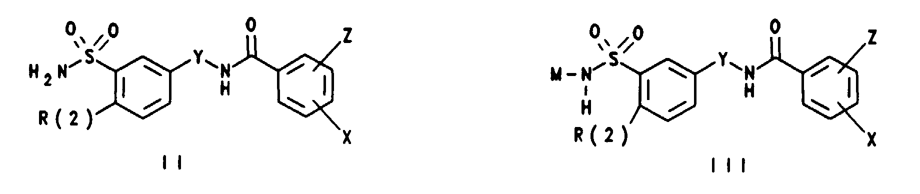 mit R(1)-substituierten Isocyanaten der Formel IV

   R(1) - N = C = O IV

   zu substituierten Benzolsulfonylharnstoffen la umsetzt.
   Als Kationen M in den Salzen der Formel III kommen Alkali- und Erdalkali-, sowie Tetraalkylammoniumionen in Betracht. Äquivalent zu den R(1)-substituierten Isocyanaten IV kann man R(1)-substituierte Carbamidsäureester, R(1)-substituierte Carbamidsäurehalogenide oder R(1)-substituierte Harnstoffe einsetzen.
(b) Unsubstituierte Benzolsulfonylharnstoffe Ia (R(1) = H) 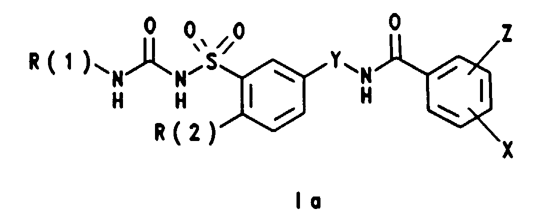 kann man durch Umsetzungen aromatischer Benzolsulfonamide der Formel II oder deren Salze III
   mit Trialkylsilylisocyanat oder Siliciumtetraisocyanat und Hydrolyse der primären siliciumsubstituierten Benzolsulfonylharnstoffe herstellen.
   Weiterhin ist es möglich, Benzolsulfonamide II oder deren Salze III durch Umsetzung mit Halogencyanen und Hydrolyse der primär entstehenden N-Cyanosulfonamide mit Mineralsäuren bei Temperaturen von 0°C bis 100°C darzustellen.
(c) Benzolsulfonylharnstoffe Ia 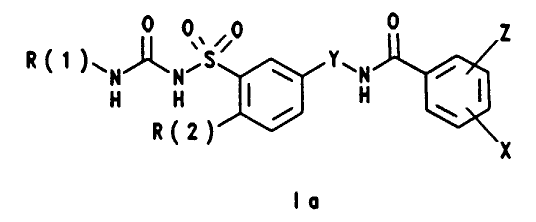 lassen sich aus aromatischen Benzolsulfonamiden II oder deren Salze III und R(1)-substituierten Trichloracetamiden der Formel V 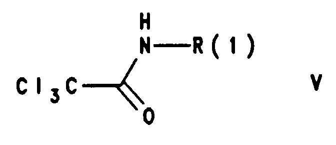 in Gegenwart einer Base in einem inerten Lösungsmittel nach Synthesis 1987, 734 - 735 bei Temperaturen von 25°C bis 150°C darstellen. Als Basen eignen sich zum Beispiel Alkalimetall- oder Erdalkalimetallhydroxide, -hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Nitrile wie Acetonitril, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(d) Benzolsulfonylthioharnstoffe Ib 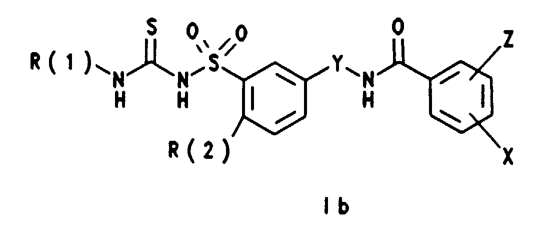 werden aus Benzolsulfonamiden II sowie deren Salze III und R(1)-substituierten Thioisocyanaten VI

   R(1) - N = C = S VI

   dargestellt.
   Unsubstituierte Benzolsulfonylthioharnstoffe Ib (R( 1) = H) 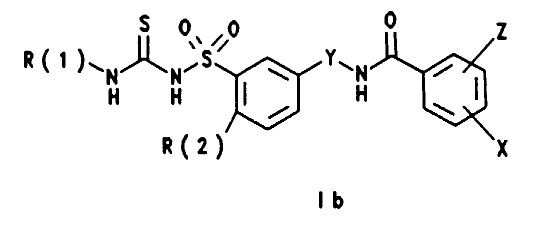 kann man durch Umsetzungen aromatischer Benzolsulfonamide II oder deren Salze III mit Trimethylsilylisothiocyanat oder Siliciumtetraisothiocyanat und Hydrolyse der primär entstehenden siliciumsubstituierten Benzolsulfonylharnstoffe herstellen.
   Weiterhin ist es möglich, aromatische Benzolsulfonamide II oder deren Salze III mit Benzoylisothiocyanat umzusetzen und die intermediären benzoyl-substituierten Benzolsulfonylthioharnstoffe mit wäßrigen Mineralsäuren zu Ib (R(1) = H) umzusetzen. Ähnliche Verfahren sind beschrieben in J. Med. Chem. 1992, 35, 1137-1144.
(e) Substituierte Benzolsulfonylharnstoffe der Formel Ia können durch Umwandlungsreaktionen von Benzolsulfonylthioharnstoffen der Struktur Ib dargestellt werden. Der Ersatz des Schwefelatoms durch ein Sauerstoffatom in den entsprechend substituierten Benzolsulfonylthioharnstoffen Ib kann man beispielsweise mit Hilfe von Oxiden oder Salzen von Schwermetallen oder auch durch Anwendung von Oxidationsmitteln wie Wasserstoffperoxid, Natriumperoxid oder Salpetersäure ausgeführt werden. Thioharnstoffe können auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Benzolsulfonylharnstoffe Ia überführt werden. Isothioharnstoffe verhalten sich bei der Entschwefelung wie Thioharnstoffe und können demzufolge ebenso als Ausgangsstoffe für diese Reaktionen dienen.
(f) Benzolsulfonylharnstoffe Ia können aus Benzolsulfonylhalogeniden der Formel VII 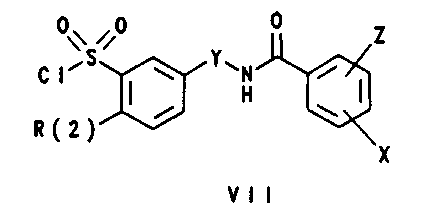 mit R(1)-substituierten Harnstoffen oder R(1)-substituierten Bis(trialkylsilyl)harnstoffen herstellen. Die Trialkylsilyl-Schutzgruppe kann aus dem resultierenden (Trialkylsilyl)benzolsulfonylharnstoff nach Standard-Methoden entfernt werden. Weiterhin kann man die Sulfonsäurechloride VII mit Parabansäuren zu Benzolsulfonylparabansäuren umsetzen, deren Hydrolyse mit Mineralsäuren die entsprechenden Benzolsulfonylharnstoffe Ia liefert.
(g) Benzolsulfonylharnstoffe Ia lassen sich durch Umsetzungen von Aminen der Formel R(1)-NH₂ mit Benzolsulfonylisocyanaten der Formel VIII 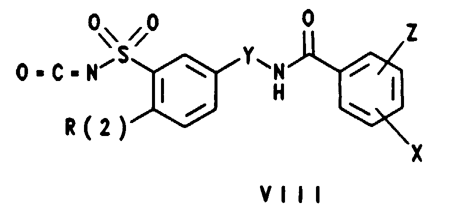 herstellen. Ebenso können Amine R(1)-NH₂ mit Benzolsulfonylcarbamidsäureestern, -carbamidsäurehalogeniden oder Benzolsulfonylharnstoffen Ia (mit R(1) = H) zu den Verbindungen Ia umgesetzt werden.
(h) Benzolsulfonylthioharnstoffe Ib lassen sich durch Umsetzungen von Aminen der Formel R(1)-NH₂ mit Benzolsulfonylisothiocyanaten der Formel IX 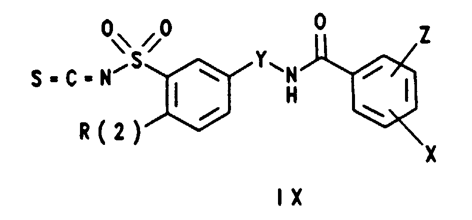 herstellen.
   Ebenso können Amine R(1)-NH₂ mit Benzolsulfonylcarbamidsäurethioestern, -carbamidsäurethiohalogeniden zu den Verbindungen Ib umgesetzt werden.
(i) Entsprechend substituierte Benzolsulfenyl- oder -sulfinylharnstoffe lassen sich mit Oxidationsmitteln, wie Wasserstoffperoxid, Natriumperoxid oder Salpetersäure, zu Benzolsulfonylharnstoffen Ia oxidieren.

Die Verbindungen I sowie deren physiologisch unbedenklichen Salze sind wertvolle Therapeutika, die sich nicht nur als Antiarrhythmika, sondern auch als Prophylaxe bei Störungen des kardiovaskulären Systems, Herzinsuffizienz, Herztranplantation oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remmington's Pharmaceutical Science, 17. Auflage, 1985, Seiten 14 - 18 Verbindungen der Formel X, 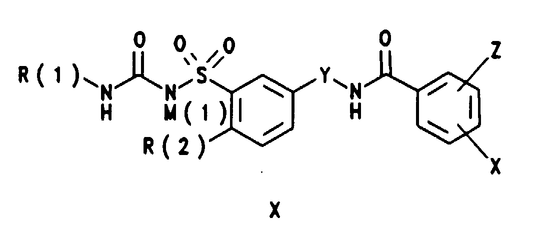 die sich aus nicht toxischen organischen und anorganischen Basen und substituierten Benzolsulfonylharnstoffen I darstellen lassen.

Bevorzugt werden hierbei Salze in denen M(1) in der Formel X Natrium-, Kalium-, Rubidium-, Calcium-, Magnesiumionen sind, sowie die Säureadditionsprodukte basischer Aminosäuren, wie zum Beispiel Lysin oder Arginin.

Die Ausgangsverbindungen für die erwähnten Syntheseverfahren der Benzolsulfonylharnstoffe I werden nach an sich bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

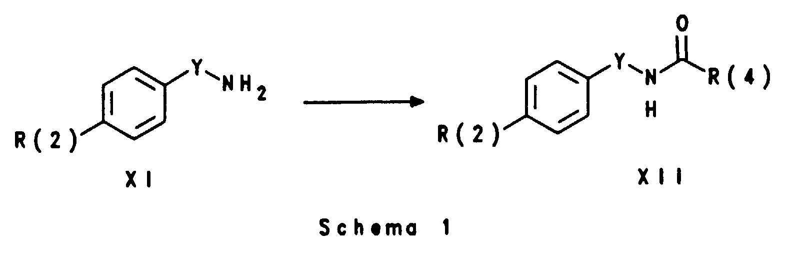

So kann man geeignet substituierte Amine der Formel Xl nach Schema 1 acylieren und einer Halogensulfonierung unterwerfen. Als acylierende Mittel für Aminogruppen eignen sich zweckmäßig die Alkylester, Halogenide (zum Beispiel Chloride oder Bromide) oder Anhydride von Carbonsäuren der Formel

R(4)-COB.

R(4) steht hierbei für einen Trihalogenmethylrest, einen (C₁-C₄)-Alkylrest oder ein Benzoesäurederivat. Das Benzoesäurederivat kann hierbei unsubstituiert oder durch ein oder zwei gleiche oder verschiedene Reste X, Z substituiert sein. Ein möglicher Substituent X steht für Wasserstoff, (C₁-C₄)-Alkyl oder Halogen, ein Substituent Z für Wasserstoff, Halogen, (C₁-C₄)-Alkyl, (C₁-C₄)-Alkoxy oder Nitro.

B ist eine Fluchtgruppe wie Halogenid, (C₁-C₄)-Alkoxy, Trihalogenacetat, (C₁-C₄)-Carboxylat. Beispiele hierfür sind Acetanhydrid, Trihalogenacetanhydrid, Acetylhalogenid, Trihalogenacetylhalogenid, Propionylchlorid, Isobutyrylbromid und -chlorid, Benzoylchlorid, 5-Chlor-2-methoxybenzoesäurechlorid oder - anhydrid sowie -(C₁-C₄)-alkylester oder 2,5-Difluor-benzoesäurechlorid. Die Synthesen der Verbindung XII werden unter Zusatz einer tertiären Base wie zum Beispiel Pyridin oder Trialkylamine in Gegenwart oder Abwesenheit eines inerten Lösungsmittels durchgeführt, wobei auch ein Katalysator wie zum Beispiel Dimethylaminopyridin zugegen sein kann. Die Umsetzung kann bei Temperaturen von etwa 0°C bis 160°C, vorzugsweise von 20 bis 150°C erreicht werden. Die Acylgruppe der Amine Xl kann sowohl eine Schutzgruppe, als auch, im Fall der Benzoesäurederivate, Teil der Verbindung I sein. Als inerte Lösungsmittel eignen sich Ether wie Tetrahydrofuran, Dioxan, Glykolether wie Ethylenglykolmonomethyl- oder monoethylether (Methylglykol oder Ethylglykol), Ethylenglykoldimethylether (Diglyme), Ketone wie Aceton oder Butanon, Nitrile wie Acetonitril, Amide wie Dimethylformamid (DMF) oder N-Methylpyrrolidon (NMP), Phosphorsäurehexamethyltriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser Lösungsmittel untereinander.

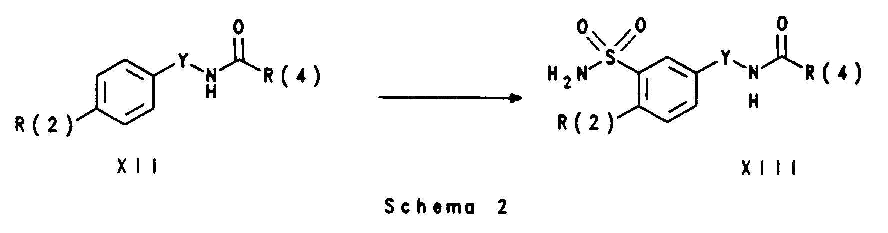

Die nach Schema 1 acylierten Amine XII können in bekannter Weise nach Schema 2 in die Sulfonamide XIII überführt werden. Die Sulfonamide XIII werden nach an sich bekannten Methoden hergestellt und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Synthesen können gewünschtenfalls in einem, zwei oder mehreren Schritten vollzogen werden. Insbesondere sind Verfahren bevorzugt, in denen das acylierte Amin XII durch elektrophile Reagenzien in An- oder Abwesenheit von inerten Lösungsmitteln bei Temperaturen von -10°C bis 120°C, vorzugsweise von 0°C bis 100°C, in aromatische Sulfonsäuren sowie deren Derivate wie zum Beispiel Sulfonsäurehalogenide überführt wird. Beispielsweise können Sulfonierungen mit Schwefelsäuren oder Oleum, Halogensulfonierungen mit Halogensulfonsäuren, Reaktionen mit Sulfurylhalogeniden in Gegenwart von wasserfreien Metallhalogeniden oder Thionylhalogeniden in Gegenwart von wasserfreien Metallhalogeniden mit anschließenden, in bekannter Weise durchgeführten Oxidationen zu aromatischen Sulfonsäurechloriden, durchgeführt werden. Sind Sulfonsäuren die primären Reaktionsprodukte, so können diese entweder direkt oder durch Behandlung mit tertiären Aminen, wie zum Beispiel Pyridin oder Trialkylaminen oder mit Alkali- oder Erdalkalihydroxiden oder Reagenzien, die diese basischen Verbindung in situ bilden, in bekannter Weise durch Säurehalogenide wie zum Beispiel Phosphortrihalogenide, Phosphorpentahalogenide, Phosphoroxychloride, Thionylhalogenide, Oxalylhalogenide, in Sulfonsäurehalogenide überführt werden. Die Überführung der Sulfonsäurederivate in Sulfonamide erfolgt in literaturbekannter Weise, vorzugsweise werden Sulfonsäurechloride in inerten Lösungsmitteln bei Temperaturen von 0°C bis 100°C mit wäßrigem Ammoniak umgesetzt. Weiterhin kann man aromatische Sulfonamide nach literaturbeschriebenen Verfahren aus den nach Schema 1 hergestellten acylierten Aminen der Formel XII durch Umsetzungen mit alkali- oder erdalkalimetallorganischen Reagenzien in inerten Lösungsmitteln und unter Inertgasatmosphäre bei Temperaturen von - 100°C bis 50°C, vorzugsweise von -100°C bis 30°C, mit Schwefeldioxid und anschließender thermischer Behandlung mit Amidosulfonsäure synthetisieren.

Fungiert die Acylgruppe als Schutzgruppe für das Amin XII, dann läßt sich diese nach Darstellung des Sulfonamids XIII mit Säuren oder Basen abspalten. Durch Spaltung mit wäßrigen Säuren oder Säuren in inerten Lösungsmitteln kann das zugehörige Säureadditionssalz entstehen. Für diese Umsetzung kommen zum Beispiel Schwefelsäure, Halogenwasserstoffsäuren, wie Chlorwasserstoffsäure oder Bromwasserstoffsäure, Phosphorsäuren wie Orthophosphorsäure, Polyphosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, zum Beispiel Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Phenylessigsäure, Citronensäure, Gluconsäure, Ascorbinsäure, Nicotinsäure, Isonicotinsäure, Methan- oder Ethansulfonsäure, Ethandisulfonsäure, 2-Hydroyethansulfonsäure, Benzolsulfonsäure, p-Toluolsulfonsäure, Napthalinmono- und -disulfonsäuren, Laurylschwefelsäure in Betracht. Die Spaltung des acylierten Amins der Formel XIII mit Basen kann auch in wäßrigen oder inerten Lösungsmitteln erfolgen. Als Basen eignen sich zum Beispiel Alkalimetall- oder Erdalkalimetallhydroxide oder auch -alkoholate in wäßrigen Medien, wie Natriumhydroxid, Kaliumhydroxid, Calciumhydroxyd, Natriumhydrid, Natriummethylat, Natriumethanolat, Kaliummethylat oder Kaliumethanolat.

Aus den so hergestellten sulfonamidsubstiuierten Aminen oder deren Säureadditionsverbindungen, werden wie oben erwähnt die aromatischen Benzolsulfonamide der Formel II hergestellt. Je nach der Natur der Glieder R(1), R(2), R(3), E, X, Y und Z wird in einzelnen Fällen das eine oder andere der genannten Verfahren für die Herstellung der Verbindungen I ungeeignet sein oder zumindest Vorkehrungen zum Schutz aktiver Gruppen notwendig machen. Derartige, verhältnismäßig selten auftretende Fälle können vom Fachmann unschwer erkannt werden, und es bereitet keine Schwierigkeiten, in solchen Fällen einen anderen der beschriebenen Synthesewege erfolgreich anzuwenden.

Die Verbindungen I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomere, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäure, Mandelsäure, Äpfelsäure oder Milchsäure. Carbinole können ferner mit Hilfe chiraler Acylierungsreagenzien, zum Beispiel R- oder S-α-Methylbenzylisocyanat amidiert und dann getrennt werden. Die verschiedenen Formen der Diastereomeren können in an sich bekannter Weise, zum Beispiel durch fraktionierte Kristallisation, getrennt und die Enantiomeren der Formel I können in an sich bekannter Weise aus den Diastereomeren in Freiheit gesetzt werden. Enantiomerentrennungen gelingen ferner durch Chromatographie an optischaktiven Trägermaterialien.

Die erfindungsgemäßen Verbindungen I und ihre physiologisch unbedenklichen Salze können zur Herstellung pharmazeutischer Zubereitungen verwendet werden. Hierbei können sie zusammen mit mindestens einem festen, flüssigen Träger oder Hilfsstoff allein oder in Kombination mit anderen Herz-Kreislaufaktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintricacetat, Gelatine, Kohlenhydrate, wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen, wie Ethanol oder Isopropanol, 1,2-Propandiol oder deren Gemische untereinander oder mit Wasser) oder Puder. Die neuen Verbindungen können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht, die Stabilisierungs- und/oder Netzmittel, Emulgatoren, Salze und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks- und/ oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von etwa mindestens 0.01, vorzugsweise 0.1 mg, insbesondere 1 mg bis höchstens 100 mg, vorzugsweise 10 mg pro kg und Tag aus, wenn Prophylaxe betrieben wird. Besonders geeignet ist ein Dosisbereich von 1 bis 10 mg pro kg und Tag. Die Dosis kann dabei als orale oder parenterale Einzeldosis oder in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Ein bevorzugter Dosisbereich in kritischen Situationen kann dann 10 bis 100 mg betragen und beispielsweise als intravenöse Dauerinfusion verabreicht werden.

Erfindungsgemäß können außer den in den Ausführungsbeispielen beschriebenen Verbindungen auch die in der folgenden Tabelle zusammengestellten Verbindungen I erhalten werden:
(1) 2-Methoxy-5-chlor-N-{5-[1-sulfonylamino-N-(methylaminocarbonyl)-2-cyclopropoxyphenyl]-ethyl}-benzamid,
(2) 2-Methoxy-5-chlor-N-{5-[1-sulfonylamino-N-(methylaminocarbonyl)-2-perfluorethoxyphenyl]-ethyl}-benzamid,
(3) 2-Methoxy-5-chlor-N-{5-[1-sulfonylamino-N-(methylaminocarbonyl)-2-(2-propoxy)phenyl]-ethyl}-benzamid,
(4) 2-Methoxy-5-chlor-N-{5-[1-sulfonylamino-N-(methylaminocarbonyl)-2-(1-propoxy)phenyl]-ethyl}-benzamid,
(5) 2-Methoxy-5-chlor-N-{5-[1-sulfonylamino-N-(trifluormethylaminocarbonyl)-2-methoxyphenyl]-ethyl}-benzamid,
(6) 2-Methoxy-5-chlor-N-{5-[1-sulfonylamino-N-(methylaminocarbonyl)-2-trifluormethoxyphenyl]-ethyl}-benzamid,
(7) 2-Ethoxy-5-chlor-N-{5-[1-sulfonylamino-N-(methylaminocarbonyl)-2-methoxyphenyl]-ethyl}-benzamid,
(8) 2-(2-Propoxy)-5-chlor-N-{5-[1-sulfonylamino-N-(methylaminocarbonyl)-2-methoxyphenyl]-ethyl}-benzamid,
(9) 2-(1-Propoxy)-5-chlor-N-{5-[1-sulfonylamino-N-(methylaminocarbonyl)-2-methoxyphenyl]-ethyl}-benzamid,
(10) 2-Cyclopropoxy-5-chlor-N-{5-[1-sulfonylamino-N-(methylaminocarbonyl)-2-methoxyphenyl]-ethyl}-benzamid,
(11) 2-Methoxy-5-fluor-N-{5-[1-sulfonylamino-N-(methylaminocarbonyl)-2-ethylphenyl]-ethyl}-benzamid,
(12) 2-Methoxy-5-fluor-N-{5-[1-sulfonylamino-N-(aminocarbonyl)-2-ethylphenyl]-ethyl}-benzamid,
(13) 2-Methoxy-5-chlor-N-{5-[1-sulfonylamino-N-(methylaminocarbonyl)-2-(1-propyl)phenyl]-ethyl}-benzamid,
(14) 2-Methoxy-5-chlor-N-{5-[1-sulfonylamino-N-(methylaminocarbonyl)-2-cyclopropylphenyl]-ethyl}-benzamid,
(15) 2-Methoxy-5-chlor-N-{5-[1-sulfonylamino-N-(aminocarbonyl)-2-cyclopropylphenyl]-ethyl}-benzamid,
(16) 2-Methoxy-5-chlor-N-{5-[1-sulfonylamino-N-(methylaminocarbonyl)-2-trifluormethylphenyl]-ethyl}-benzamid,
(17) 2-Methoxy-5-fluor-N-{5-[1-sulfonylamino-N-<methylaminocarbonyl)-2-cyclopropoxyphenyl]-[(1)-(R)-1-methylethyl]}-benzamid,
(18) 2-Methoxy-5-fluor-N-{5-[1-sulfonylamino-N-(methylaminocarbonyl)-2-cyclopropoxyphenyl]-[(1)-(S)-1-methylethyl]}-benzamid,
(19) 2-Methoxy-5-fluor-N-{5-[1-sulfonylamino-N-(methylaminocarbonyl)-2-cyclopropoxyphenyl]-[(2)-(R)-2-methylethyl]}-benzamid,
(20) 2-Methoxy-5-fluor-N-{5-[1-sulfonylamino-N-(methylaminocarbonyl)-2-cyclopropoxyphenyl]-[(2)-(S)-2-methylethyl]}-benzamid,
(21) 2-Methoxy-5-chlor-N-{5-[1-sulfonylamino-N-(methylaminothiocarbonyl)-2-cyclopropoxyphenyl]-[(1)-(R)-1-methylethyl]}-benzamid,
(22) 2-Methoxy-5-fluor-N-{5-[1-sulfonylamino-N-(methylaminothiocarbonyl)-2-cyclopropoxyphenyl]-[(1)-(S)-1-methylethyl]}-benzamid,
(23) 2-Methoxy-5-chlor-N-{5-[1-sulfonylamino-N-(methylaminothiocarbonyl)-2-cyclopropoxyphenyl]-[(2)-(R)-2-methylethyl]}-benzamid,
(24) 2-Methoxy-5-fluor-N-{5-[1-sulfonylamino-N-(methylaminothiocarbonyl)-2-cyclopropoxyphenyl]-[(2)-(S)-2-methylethyl]}-benzamid,
(25) 2-Methoxy-5-fluor-N-{5-[1-sulfonylamino-N-(methylaminocarbonyl)-2-methylphenyl]-[(1)-(R)-1-methylethyl]}-benzamid, (26) 2-Methoxy-5-fluor-N-{5-[1-sulfonylamino-N-(methylaminocarbonyl)-2-methylphenyl]-[(1)-(S)-1-methylethyl]}-benzamid,
(27) 2-Methoxy-5-fluor-N-{5-[1-sulfonylamino-N-(methylaminocarbonyl)-2-methylphenyl]-[(2)-(R)-2-methylethyl]}-benzamid,
(28) 2-Methoxy-5-fluor-N-{5-[1-sulfonylamino-N-(methylaminocarbonyl)-2-methylphenyl]-[(2)-(S)-2-methylethyl]}-benzamid,
(29) 2-Methoxy-5-chlor-N-{5-[1-sulfonylamino-N-(methylaminothiocarbonyl)-2-methylphenyl]-[(1)-(R)-1-methylethyl]}-benzamid,
(30) 2-Methoxy-5-fluor-N-{5-[1-sulfonylamino-N-(methylaminothiocarbonyl)-2-methylphenyl]-[(1)-(S)-1-methylethyl]}-benzamid,
(31) 2-Methoxy-5-chlor-N-{5-[1-sulfonylamino-N-(methylaminothiocarbonyl)-2-methylphenyl]-[(2)-(R)-2-methylethyl]}-benzamid,
(32) 2-Methoxy-5-fluor-N-{5-[1-sulfonylamino-N-(methylaminothiocarbonyl)-2-methylphenyl]-[(2)-(S)-2-methylethyl]}-benzamid,
(33) 2-Methoxy-5-fluor-N-{5-[1-sulfonylamino-N-(methylaminocarbonyl)-2-methoxyphenyl]-[(1)-(R)-1-methylethyl]}-benzamid,
(34) 2-Methoxy-5-fluor-N-{5-[1-sulfonylamino-N-(methylaminocarbonyl)-2-methoxyphenyl]-[(1)-(S)-1-methylethyl]}-benzamid,
(35) 2-Methoxy-5-fluor-N-{5-[1-sulfonylamino-N-(methylaminocarbonyl)-2-methoxyphenyl]-[(2)-(R)-2-methylethyl]}-benzamid,
(36) 2-Methoxy-5-fluor-N-{5-[1-sulfonylamino-N-(methylaminocarbonyl)-2-methoxyphenyl]-[(2)-(S)-2-methylethyl]}-benzamid,
(37) 2-Methoxy-5-chlor-N-{5-[1-sulfonylamino-N-(methylaminothiocarbonyl)-2-methoxyphenyl]-[(1)-(R)-1-methylethyl]}-benzamid,
(38) 2-Methoxy-5-fluor-N-{5-[1-sulfonylamino-N-(methylaminothiocarbonyl)-2-methoxyphenyl]-[(1)-(S)-1-methylethyl]}-benzamid,
(39) 2-Methoxy-5-chlor-N-{5-[1-sulfonylamino-N-(methylaminothiocarbonyl)-2-methoxyphenyl]-[(2)-(R)-2-methylethyl]}-benzamid,
(40) 2-Methoxy-5-fluor-N-{5-[1-sulfonylamino-N-(methylaminothiocarbonyl)-2-methoxyphenyl]-[(2)-(S)-2-methylethyl]}-benzamid,
(41) 2-Methoxy-5-chlor-N-{5-[1-sulfonylamino-N-(methylaminocarbonyl)-2-methoxyphenyl]-(3-propyl)}-benzamid,
(42) 2-Methoxy-5-chlor-N-{5-[1-sulfonylamino-N-(methylaminocarbonyl)-2-methoxyphenyl]-(4-butyl)}-benzamid,
(43) 2-Methoxy-5-fluor-N-{5-[1-sulfonylamino-N-(methylaminothiocarbonyl)-2-methoxyphenyl]-(3-propyl)}-benzamid,
(44) 2-Methoxy-5-fluor-N-{5-[1-sulfonylamino-N-(methylaminothiocarbonyl)-2-methoxyphenyl]-(4-butyl)}-benzamid.

### Beispiel 1:

2-Methoxy-5-chlor-N-{5-[1-sulfonylamino-N-(methylaminocarbonyl)-2-methoxyphenyl]-ethyl}-benzamid: 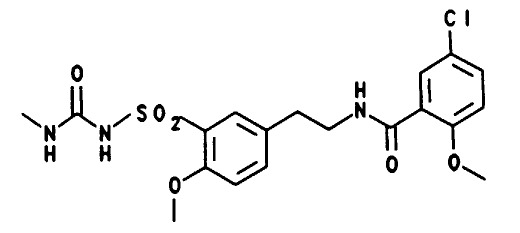 0.30 g (0.8 mmol) 2-Methoxy-5-chlor-N-[5-(-1-sulfonylamino-2-methoxyphenyl)-ethyl]-benzamid werden in 2 ml trockenem DMSO gelöst und nach Zugabe von 0.9 g (2.23 mmol) Natriumhydroxid und 0.15 g (0.8 mmol) N-Methyltrichloracetamid 3 h bei 80°C erhitzt. Der kühle Reaktionsansatz wird auf wäßrige, verdünnte Salzsäure gegossen, der Niederschlag abgesaugt und aus Acetonitril umkristallisiert. 2-Methoxy-5-chlor-N-{5-[1-sulfonylamino-N-(methylaminocarbonyl)-2-methoxyphenyl]-ethyl}-benzamid schmilzt bei 201-203°C.

### Herstellung der Ausgangsverbindung:

1.51 g (10.0 mmol) 4-Methoxy-β-phenylethylamin werden in 40 ml Pyridin gelöst, mit einer Spatelspitze Dimethylaminopyridin versetzt und mit einer Lösung von 2.15 g (10.5 mmol) 2-Methoxy-5-chlorbenzoesäurechlorid versetzt. Die Reaktionsmischung wird auf kalte verdünnte Salzsäure gegossen, das ausgefallene Produkt abgesaugt und getrocknet. Man erhält 4-Methoxy-β-ethyl-(2-methoxy-5-chlorbenzamid) als farblose Kristalle vom Schmelzpunkt 83-84°C. Das so erhaltene Benzamid wird in kalte Chlorsulfonsäure eingetragen. Nach vollständiger Umsetzung gießt man die Reaktionsmischung auf Eis, saugt ab (Schmelzpunkt des Sulfonsäurechlorids: 140-141°C) und löst den Niederschlag in Aceton. Diese Lösung wird mit überschüssigem, konzentriertem, wässrigem Ammoniak versetzt und nach Abklingen der exothermen Reaktion auf ein Drittel des ursprünglichen Volumens eingeengt. Das 2-Methoxy-5-chlor-N-[5-(-1-sulfonylamino-2-methoxyphenyl)-ethyl]-benzamid fällt als farblose Kristalle vom Schmelzpunkt 220-222°C an.

### Beispiel 2:

2-Methoxy-N-{5-[1-sulfonylamino-N-(methylaminocarbonyl)-2-methoxyphenyl]-ethyl}-benzamid: 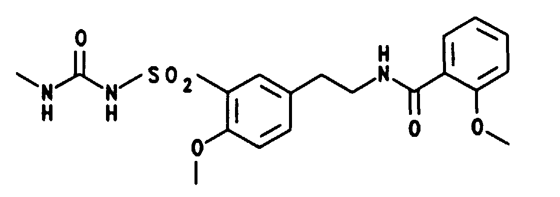 0.30 g (6.6 mmol) 2-Methoxy-5-chlor-N-{5-[1-sulfonylamino-N-(methylaminocarbonyl)-2-methoxyphenyl]-ethyl}-benzamid werden in 20 ml Methanol gelöst und mit 0.1 g 10proz. Palladium auf Aktivkohle 24 h in einer Wasserstoffatmosphäre gerührt. Der Katalysator wird abfiltriert, das Lösungsmittel entfernt und der farblose Rückstand in Acetonitril umkristallisiert. Schmp.: 190-191°C.

### Beispiel 3:

2-Methoxy-5-chlor-N-{3-[1-sulfonylamino-N-(methylaminocarbonyl)phenyl]-ethyl}-benzamid 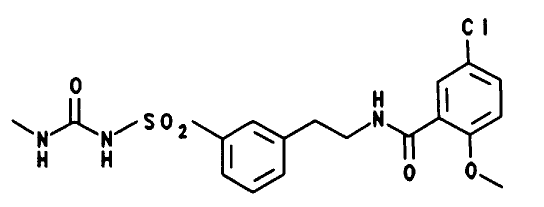 0.40 g (1.0 mmol) 2-Methoxy-5-chlor-N-[3-(-1-sulfonylaminophenyl)-ethyl]-benzamid werden in 5 ml trockenem DMF gelöst und nach Zugabe von 0.10 g (2.5 mmol) Natriumhydroxid und 0.27 g (1.2 mmol) N-Methyltrichloracetamid 2 h bei 80°C erhitzt. Der kühle Reaktionsansatz wird auf wäßrige, verdünnte Salzsäure gegossen, der Niederschlag abgesaugt und aus Acetonitril umkristallisiert. 2-Methoxy-5-chlor-N-{3-[1-sulfonylamino-N-(methylaminocarbonyl)phenyl]-ethyl}-benzamid schmilzt bei 179-180°C.

### Herstellung der Ausgangsverbindung:

15.6 g (0.1 mol) 2-(4-Chlorphenyl)ethylamin werden in 80 ml Tetrahydrofuran und 16.3 ml (0.15 mol) Pyridin gelöst und unter Kühlung mit 21.2 ml (0.15 mol) Trifluoracetanhydrid versetzt. Nach 1 bis 2 Stunden wird die Reaktionsmischung auf Eis gegossen und das ausgefallene Produkt abgesaugt. Dieses Produkt wird wie in Beispiel 1 beschrieben in das entsprechende Sulfonamid überführt (Schmp.: 172-174°C). Reduktion des chlorierten Sulfonamids mittels Wasserstoff in Gegenwart von 10proz. Palladium auf Aktivkohle in Methanol als Lösungsmittel liefert 2-(1-Sulfonylaminophenyl)ethyltrifluoracetamid, daß durch Erhitzen in wäßriger Salzsäure in das entsprechende Aminhydrochlorid überführt wird. Letzteres wird mit 2-Methoxy-5-chlorbenzoesäurechlorid und Triethylamin in Dimethylformamid und in Gegenwart von Dimethylaminopyridin zu 2-Methoxy-5-chlor-N-[3-(-1-sulfonylaminophenyl)-ethyl]-benzamid umgesetzt. Schmp.: 196-198°C.

### Beispiel 4:

2-Methoxy-5-chlor-N-{5-[1-sulfonylamino-N-(methylaminocarbonyl)-2-methylphenyl]-ethyl}-benzamid. 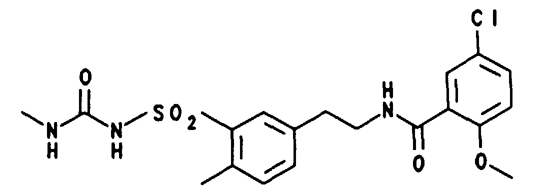 2-Methoxy-5-chlor-N-{5-[1-sulfonylamino-N-(methylaminocarbonyl)-2-methylphenyl]-ethyl}-benzamid wird aus p-Tolyl-β-ethylamin analog der in Beispiel 1 beschriebenen Weise hergestellt. Schmp.: 192-193°C.

### Beispiel 5:

2-Methoxy-5-chlor-N-{5-[1-sulfonylamino-N-(methylaminocarbonyl)-2-(2-propyl)-phenyl]-ethyl}-benzamid. 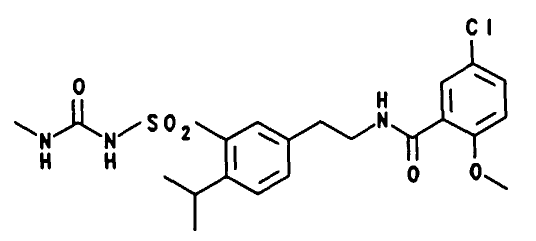 2-Methoxy-5-chlor-N-{5-[1-sulfonylamino-N-(methylaminocarbonyl)-2-(2-propyl)-phenyl]-ethyl}-benzamid läßt sich aus 2-[2-(Propyl)phenyl]ethylamin analog Beispiell 1 darstellen und schmilzt bei 190°C. Bei der Chlorsulfonierung von 4-Cumyl-β-ethyl-(2-methoxy-5-chlorbenzamid) entstehen isomere Sulfonsäurechloride, die auf der folgenden Stufe des Sulfonamids durch Kristallisation aus Ethylacetat getrennt werden.

### Beispiel 6:

2,5-Difluor-N-{5-[1-sulfonylamino-N-(methylaminocarbonyl)-2-methylphenyl]-ethyl}-benzamid 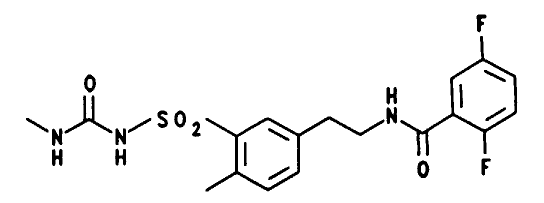 2-(1-Sulfonylamino-2-methylphenyl)ethylamin Hydrochlorid, daß in Anlehnung an Beispiel 4 aus 2-(p-Tolyl)ethylamin synthetisiert wird, kann wie beschrieben zunächst mit 2,5-Difluorbenzoesäurechlorid, anschließend mit N-Methyltrichloracetamid und Natriumhydroxid in DMSO in 2,5-Difluor-N-{5-[1-sulfonylamino-N-(methylaminocarbonyl)-2-methylphenyl]-ethyl}-benzamid umgesetzt werden Schmp.: 196-198°C.

### Beispiel 7:

2-Nitro-5-chlor-N-{5-[1-sulfonylamino-N-(methylaminocarbonyl)-2-methylphenyl]-ethyl}-benzamid 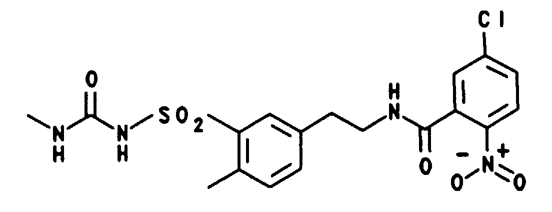 Analog Beispiel 6 kann 2-Nitro-5-chlor-N-{5-[1-sulfonylamino-N-(methylaminocarbonyl)-2-methylphenyl]-ethyl}-benzamid hergestellt werden, das zwischen 165 und 170°C unter Zersetzung schmilzt.

### Beispiel 8:

2-Methoxy-5-chlor-N-[1-sulfonylamino-N-(methylaminocarbonyl)-2-methoxybenzyl]-benzamid. 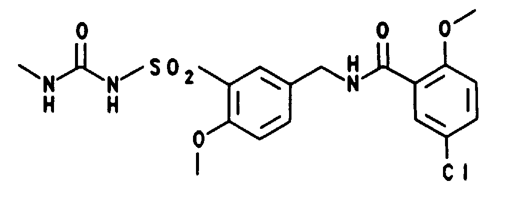 4-Methoxybenzylamin wird wie in Beispiel 1 beschrieben zu 2-Methoxy-5-chlor-N-[1-sulfonylamino-N-(methylaminocarbonyl)-2-methoxybenzyl]-benzamid umgesetzt. Die Verbindung ist farblos, kristallin und schmilzt im Temperaturintervall von 206-210°C.

### Beispiel 9:

2-Methoxy-5-chlor-N-{5-[1-sulfonylamino-N-(aminocarbonyl)-2-methoxyphenyl]-ethyl}-benzamid 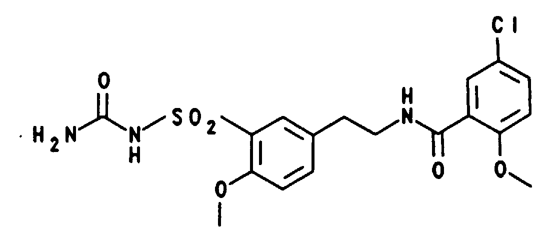 0.40 g (1.0 mmol) 2-Methoxy-5-chlor-N-[5-(-1-sulfonylamino-2-methoxyphenyl)-ethyl]-benzamid aus Beispiel 1 werden in 5 ml Acetonitril gelöst und mit 0.14 g (1.0 mmol) Kaliumcarbonat sowie 1 ml 1 molarer Bromcyanlösung in Acetonitril versetzt. Nach mehrstündigem Erhitzen isoliert man säulenchromatographisch 0.14g 2-Methoxy-5-chlor-N-[5-(-1-sulfonylamino-N-cyano-2-methoxyphenyl)-ethyl]-benzamid, das mit kalter, halbkonzentrierter Schwefelsäure in 2-Methoxy-5-chlor-N-{5-[1-sulfonylamino-N-(aminocarbonyl)-2-methoxyphenyl]-ethyl}-benzamid überführt wird. Schmp.: 180-185°C.

### Beispiel 10:

2-Methoxy-5-chlor-N-{5-[1-sulfonylamino-N-(methylaminothiocarbonyl)-2-methoxyphenyl]-ethyl}-benzamid. 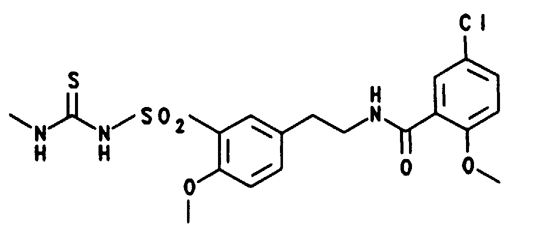 0.40 g (1.0 mmol) 2-Methoxy-5-chlor-N-[5-(-1-sulfonylamino-2-methoxyphenyl)-ethyl]-benzamid aus Beispiel 1 wird unter Argon in 5 ml trockenem DMF gelöst und bei 0°C mit 42 mg Natriumhydrid (60%ige Dispersion in Weißöl) versetzt. Das Kältebad wird entfernt und die Reaktionsmischung 30 min bei Raumtemp. nachgerührt. In die Lösung des Natriumsulfonamids gibt man 0.10 g Methylisothiocyanat und rührt 5 h bei Raumtemp. sowie 1 h bei 70°C nach. Nach dem Abkühlen gießt man die Reaktionsmischung auf 50 ml 0.5_{N} Salzsäure. Das ausgefallene Produkt wird abgesaugt und getrocknet. Ausbeute: 96%, Schmp.: 190-193°C.

### Beispiel 11:

2-Methoxy-5-chlor-N-{5-[1-sulfonylamino-N-(methylaminocarbonyl)-2-ethoxyphenyl]-ethyl}-benzamid, 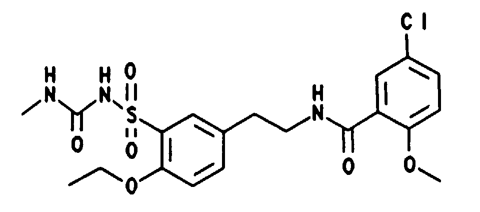 2-Methoxy-5-chlor-N-{5-[1-sulfonylamino-N-(methylaminocarbonyl)-2-ethoxyphenyl]-ethyl)-benzamid wird analog zu Beispiel 1 aus 4-Ethylphenyl-β-ethylamin dargestellt. Schmp.: 190-195°C.

### Beispiel 12:

2-Methoxy-5-chlor-N-{5-[1-sulfonylamino-N-(methylaminocarbonyl)-2-ethylphenyl]-ethyl}-benzamid, 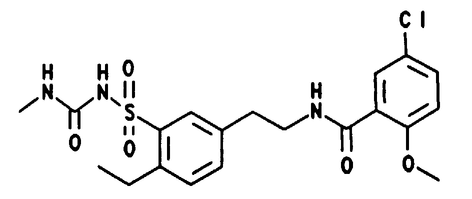 2-Methoxy-5-chlor-N-{5-[1-sulfonylamino-N-(methylaminocarbonyl)-2-ethylphenyl]-ethyl}-benzamid wird in Anlehnung an Beispiel 1 aus 4-Ethylphenyl-β-ethylamin synthetisiert. Schmp.: 207°C.

### Beispiel 13:

2-Methoxy-5-chlor-N-{5-[1-sulfonylamino-N-(methylaminocarbonyl)-2-methoxyphenyl]-(3-propyl)}-benzamid, 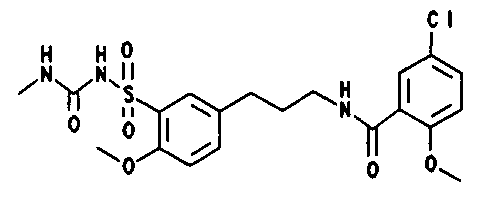 2-Methoxy-5-chlor-N-{5-[1-sulfonylamino-N-(methylaminocarbonyl)-2-methoxyphenyl]-(3-propyl)}-benzamid wird analog zu Beispiel 1 aus 4-Methoxyphenyl-γ-propylamin synthetisiert. Schmp.: 285°C.

### Beispiel 14:

2-Methoxy-5-chlor-N-{5-[1-sulfonylamino-N-(methylaminocarbonyl)-2-methoxyphenyl]-(4-butyl)}-benzamid, 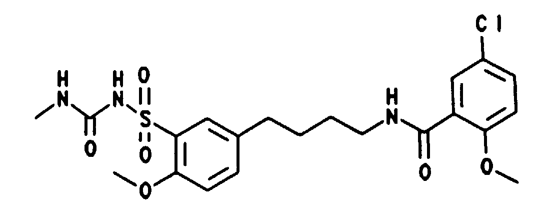 Die Umsetzung von 4-Methoxyphenyl-6-butylamin gemäß Beispiel 1 liefert 2-Methoxy-5-chlor-N-{5-1-sulfonylamino-N-(methylaminocarbonyl)-2-methoxyphenyl]-(4-butyl)}-benzamid. Die Verbindung schmilzt bei 188-190°C.

### Beispiel 15:

2-Methoxy-5-chlor-N-{5-[1-sulfonylamino-N-(methylaminothiocarbonyl)-2-methylphenyl]-ethyl}-benzamid, 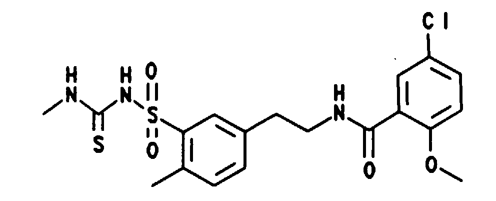 2-Methoxy-5-chlor-N-{5-[1-sulfonylamino-N-(methylaminothiocarbonyl)-2-methylphenyl]-ethyl}-benzamid wird analog zu Beispiel 10 aus 2-Methoxy-5-chlor-N-[5-(1-sulfonylamino-2-methylphenyl)-ethyl]-benzamid und Methylisothiocyanat synthetisiert. Schmp.: 183°C.

### Beispiel 16:

2-Methoxy-5-fluor-N-{5-[1-sulfonylamino-N-(methylaminocarbonyl)-2-methoxyphenyl]-ethyl}-benzamid 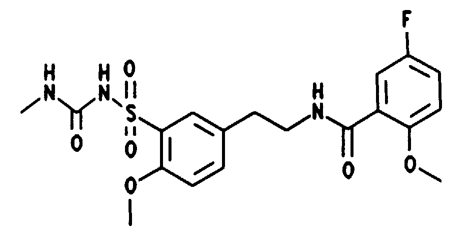 2-Methoxy-5-fluor-N-{5-(1-sulfonylamino-N-(methylaminocarbonyl)-2-methoxyphenyl]-ethyl}-benzamid wird analog zu Beispiel 1 synthetisiert. Schmp.: 199-200°C.

### Beispiel 17:

2-Methoxy-5-fluor-N-{5-[1-sulfonylamino-N-(methylaminothiocarbonyl)-2-methoxyphenyl]-ethyl}-benzamid 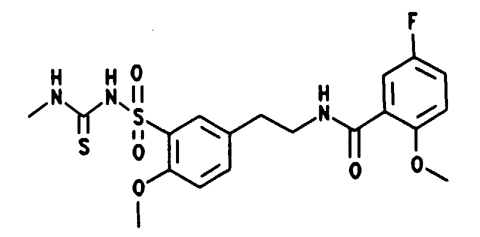 2-Methoxy-5-fluor-N-{5-[1-sulfonylamino-N-(methylaminothiocarbonyl)-2-methoxyphenyl]-ethyl}-benzamid wird in Anlehnung an Beispiel 10 dargestellt. Schmp.: 182-185°C.

### Beispiel 18:

2-Methoxy-5-chlor-N-{5-[1-sulfonylamino-N-(methylaminothiocarbonyl)-2-ethylphenyl]-ethyl}-benzamid 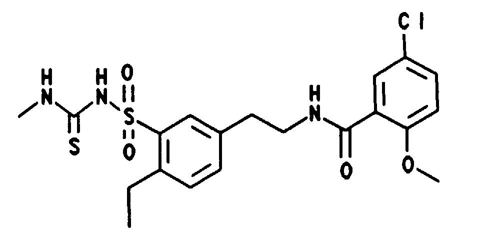 Die Umsetzung von 2-Methoxy-5-chlor-N-[5-(1-sulfonylamino-2-ethylphenyl)-ethyl]-benzamid und Methylisocyanat nach Beispiel 10 liefert 2-Methoxy-5-chlor-N-{5-[1-sulfonylamino-N-(methylaminothiocarbonyl)-2-ethylphenyl]-ethyl}-benzamid.
Schmp.: 155°C.

### Beispiel 19

2-Methoxy-5-chlor-N-{5-[-1-sulfonylamino-N-(methylaminothiocarbonyl)-2-chlorophenyl]-ethyl}-benzamid: 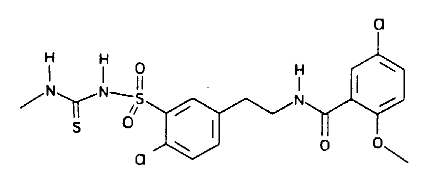 Diese Verbindung wurde nach dem Verfahren von Beispiel 10 erhalten, ausgehend von 2-Methoxy-5-chlor-N-{5-[1-sulfonylamino-2-chlorophenyl]-ethyl}-benzamid und Methylisothiocyanat.
Schmelzpunkt 194 - 196°C.

### Beispiel 20

2-Methoxy-5-chlor-N-{5-[1-sulfonylamino-N-(methylaminothiocarbonyl)-phenyl]-ethyl}-benzamid: 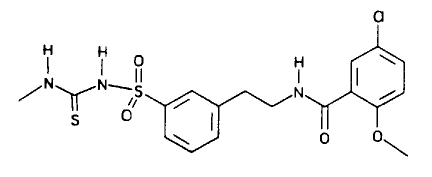 Die Titelverbindung wurde nach dem Verfahren von Beispiel 10 erhalten, ausgehend von 2-Methoxy-5-chlor-N-{5-[1-sulfonylamino-phenyl]-ethyl}-benzamid und Methylisothiocyanat.
Schmelzpunkt 173 - 175°C.

### Beispiel 21

2-Methoxy-5-chlor-N-{5-[1-sulfonylamino-N-(methylaminothiocarbonyl)-2-ethoxyphenyl]-ethyl}-benzamid: 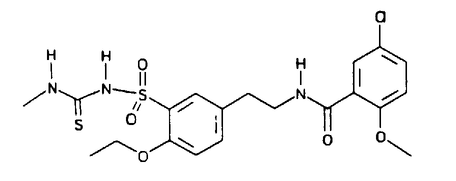 Die Titelverbindung wurde nach dem Verfahren von Beispiel 10 erhalten, ausgehend von 2-Methoxy-5-chlor-N-(5-[1-sulfonylamino-2-ethoxyphenyl]-ethyl}-benzamid und Methylisothiocyanat.
Schmelzpunkt 185 - 187°C.

### Beispiel 22

2-Methoxy-5-chlor-N-{5-[1-sulfonylamino-N-(methylaminothiocarbonyl)-2-(2,2,2-trifluorethoxy)phenyl]-ethyl}-benzamid: 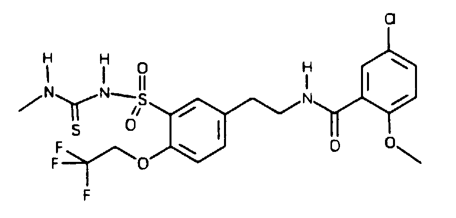 Die Titelverbindung wurde nach dem Verfahren von Beispiel 10 erhalten, ausgehend von 2-Methoxy-5-chlor-N-{5-[1-sulfonylamino-2-(2,2,2-trifluorethoxy)phenyl]-ethyl}-benzamid und Methylisothiocyanat.
Schmelzpunkt 167 - 170°C.

### Pharmakologische Daten:

Mit den folgenden Modellen können die therapeutischen Eigenschaften der Verbindungen I nachgewiesen werden:

### (1) Aktionspotentialdauer am Papillarmuskel des Meerschweinchens:

(a) Einleitung
ATP-Mangelzustände, wie sie während einer Ischämie in der Herzmuskelzelle beobachtet werden, führen zu einer Verkürzung der Aktionspotentialdauer. Sie gelten als eine der Ursachen für sogenannte Reentryarrhythmien, die den plötzlichen Herztod verursachen können. Die Öffnung von ATP-sensitiven K-Kanälen durch das Absinken von ATP gilt hierfür als ursächlich.
(b) Methode
Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCl, 0.048% KCl, 0.024% CaCl₂, 0.02% NaHCO₃, und 0.1% Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2·10⁻⁵ Mol pro Liter zugesetzt. Das Aktionspotential wird mit einem Amplifier von Hugo Sachs verstärkt auf einem Oszilloskop dargestellt. Die Dauer des Aktionspotentials wird bei einem Repolarisierungsgrad von 95% (APD₉₅) bestimmt. Aktionspotentialverkürzungen werden entweder durch Zugabe einer 1 µM starken Lösung des Kaliumkanalöffners Hoe 234 (Rilmakalim) [W. Linz, E. Klaus, U. Albus, R.H.A. Becker, D. Mania, H.C.Englert, B.A. Schölkens Arzneimittelforschung/Drug Research, Band 42 (II), 1992, S. 1180 - 1185] oder durch Zugabe von 2-Desoxyglucose (DEO) hervorgerufen. Durch 2-Desoxyglucose werden in der experimentellen Physiologie ATP-Mangelzustände durch Blockade des Glucosestoffwechsels hervorgerufen. Der aktionspotential-verkürzende Effekt dieser Substanzen wurde durch die gleichzeitige Gabe der Testsubstanzen verhindert oder veringert. Testsubstanzen wurden als Stammlösungen in Propandiol der Badlösung zugesetzt. Die angegebenen Werte beziehen sich auf Messungen 30 min nach der Zugabe. Als Kontrolle gilt die APD₉₅ in Gegenwart von DEO oder HOE 234 und in Abwesenheit der Testsubstanz.
(c) Resultate:
Folgende Werte wurden gemessen:.

| Messung | APD₉₅-DEO^{a)} [ms] | APD₉₅-HOE 234^{a)} [ms] |
|---|---|---|
| Kontrolle | < 40 | < 40 |
| Beispiel 1 | 107 ± 14 (155 ± 9) n = 3 | 138 ± 3 (160 ± 20) n = 3 |
| Beispiel 4 | 110 ± 23 (180 ± 5) n = 3 | 123 ± 15 (172 ± 18) n = 3 |
| Beispiel 10 | 125 (175) n = 1 | 137 ± 20 (150 ± 23) n = 3 |

| | | |
|---|---|---|
| ^{a)} den Meßwerten aus n-Versuchen sind die korrespondierenden Leerwerte in Klammern nachgestellt. Die Leerwerte sind die APD₉₅-Werte zu Versuchsbeginn ohne DEO, HOE 234 und Testsubstanz in der Ringerlösung. | | |

### (2) Membranpotential an isolierten β-Zellen:

(a) Einleitung
Der Wirkmechanismus der blutzuckersenkenden Sulfonylharnstoffe ist in groben Zügen aufgeklärt. Zielorgan sind die β-Zellen des Pankreas wo es zu einer Mehrausschüttung des blutzuckersenkenden Hormons Insulin kommt. Die Freisetzung von Insulin wird über das Zellmembranpotential gesteuert. Glibenclamid bewirkt eine Depolarisation der Zellmembran, was über einen vermehrten Einstrom von Calciumionen die Insulinfreisetzung fördert. Das Ausmaß dieser Depolarisation der Zellmembran △U wurde an RINm5F Zellen, einer Pankreastumorzellinie, für einige der erfindungsgemäßen Verbindungen bestimmt. Die Wirkstärke einer Verbindung in diesem Modell sagt das Ausmaß des blutzuckersenkenden Potentials dieser Verbindung voraus.
(b) Methode
Zellkultur von RINm5F Zellen
RINm5F Zellen wurden in RPMI 1640 Kulturmedium (Flow), dem 11 mmol Glukose, 10% (vol/vol) fötales Kälberserum, 2 mmol Glutamin und 50 µg/ml Gentamycin zugesetzt wurde, bei 37°C kultiviert. Für die Untersuchungen wurden die Zellen durch Inkubation (ca. 3 min) in einem Ca²⁺-freien Medium, das 0.25% Trypsin enthielt, isoliert und auf Eis aufbewahrt.
Meßmethode
Isolierte RINm5F Zellen wurden in eine Plexiglaskammer auf einem inversen Mikroskop, das mit Differential-Interferenz-Kontrast Optik ausgerüstet ist, gebracht. Unter optischer Kontrolle (400-fache Vergrößerung) wurde eine feuerpolierte Mikropipette mit Öffungsdurchmessser von etwa 1 µm mit Hilfe eines Mikromanipulators auf die Zelle aufgesetzt. Durch Anlegen eines leichten Unterdruckes in der Patch-Pipette wurde zunächst eine hohe elektrische Abdichtung zwischen Glas und Zellmembran hergestellt und anschließend durch Erhöhung des Unterdruckes der Membranfleck unter der Meßpipette aufgerissen. In dieser Ganzzellenkonfiguration wurde mit Hilfe eines Patch-Clamp Verstärkers (L/M EPC 7) das Zellpotential registriert und durch Anlegen einer Spannungsrampe der Ganzzellenstrom gemessen.
Lösungen: Die Patch-Pipette war mit KCl-Lösung gefüllt (in mmol): 140 KCl, 10 NaCl, 1.1 MgCl₂, 0.5 EGTA, 1 Mg-ATP, 10 HEPES, pH = 7.2, und im Bad befand sich NaCl-Lösung (in mmol): 140 NaCI, 4.7 KCI, 1.1 MgCl₂, 2 CaCl₂, 10 HEPES, pH = 7.4. Von den Testsubstanzen wurden Stocklösungen (Konzentration 100 mmol) in Dimethylsulfoxid (DMSO) und entsprechende Verdünnungen in NaCl-Lösung hergestellt. DMSO alleine hatte keinen Effekt auf das Zellpotential. Um das Zellpotential unter Kontrollbedingungen zu stabilisieren, wurde der Öffner für ATP-sensitive K⁺ Kanäle Diazoxid (100 µmol) bei allen Versuchen in die Badlösung zugegeben. Alle Experimente wurden bei 34 ± 1°C durchgeführt.
(c) Ergebnisse (Die Konzentrationen der erfindungsgemäßen
Verbindungen betragen in den Versuchen 10⁻⁵ Mol pro Liter)

| Messung | △U (mv)^{a)} |
|---|---|
| Beispiel 1 | 13 (-76) n = 6 |
| Beispiel 4 | 19 (-76) n = 3 |
| Beispiel 10 | 11 (-79) n = 3 |

| | |
|---|---|
| ^{a)} den Meßwerten aus n-Versuchen sind die korrespondierenden Leerwerte in Klammern nachgestellt. Die Leerwerte sind die Zellpotentiale unter Diazoxid-Gabe. | |

## Patentansprüche

1. Substituierte Benzolsulfonylharnstoffe und -thioharnstoffe der Formel I 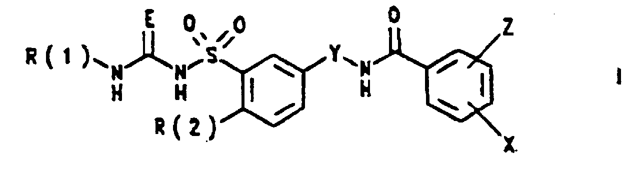 worin
R(1) für Wasserstoff, Methyl, CH₂F, CHF₂ oder Trifluormethyl steht,
R(2) Wasserstoff, F, Cl, Br, l, (C₁-C₆)-Alkyl, (C₁-C₆)-Alkoxy, (C₁-C₆)-Mercaptoalkyl, (C₁-C₆)-Fluoralkoxy oder (C₁-C₆)-Fluoralkyl ist,
E für Sauerstoff oder Schwefel steht,
Y eine Kohlenwasserstoffkette der Formel:
-[CR(3)₂]ₙ-
mit R(3) = H oder (C₁-C₂)-Alkyl und n = 1, 2, 3 oder 4 ist,
X für Wasserstoff, F, Cl, Br, J oder (C₁-C₆)-Alkyl steht,
Z für F, Cl, Br, l, NO₂, (C₁-C₄)-Alkoxy oder (C₁-C₄)-Alkyl steht,
und ihre physiologisch unbedenklichen Salze.

2. Verbindung der Formel I nach Anspruch 1, dadurch gekennzeichnet, daß darin bedeuten:
R(1) Wasserstoff, Methyl, Trifluormethyl,
R(2) Wasserstoff, (C₁-C₆)-Alkyl, (C₁-C₆)-Alkoxy, (C₁-C₆)-Mercaptoalkyl, (C₁-C₆)-Fluoralkyl, (C₁-C₆)-Fluoralkoxy, F, Cl, Br oder I,
E Sauerstoff oder Schwefel,
Y eine Kohlenwasserstoffkette der Formel
-[CR(3)₂]ₙ-
mit R(3) = H oder (C₁-C₂)-Alkyl und n = 1, 2, 3 oder 4,
X Wasserstoff, F, Cl oder (C₁-C₄)-Alkyl,
Z Nitro, F, Cl, (C₁-C₄)-Alkyl oder (C₁-C₄)-Alkoxy.

3. Verbindung der Formel I nach Anspruch 1, dadurch gekennzeichnet, daß darin bedeuten:
R(1) Wasserstoff, Methyl, und
R(2) Wasserstoff, Fluor oder Chlor,
E Sauerstoff oder Schwefel,
Y eine Kohlenwasserstoffkette der Formel
-[CR(3)₂]ₙ-
mit R(3) = H oder (C₁-C₂)-Alkyl und n = 1, 2, 3 oder 4,
X Wasserstoff, F, Cl, (C₁-C₄)-Alkyl,
Z Chlor, Fluor, (C₁-C₄)-Alkyl, (C₁-C₄)-Alkoxy.

4. Verbindung der Formel I nach Anspruch 1, dadurch gekennzeichnet, daß darin bedeuten:
R(1) Wasserstoff, Methyl,
R(2) (C₁-C₆)-Fluoralkyl, (C₁-C₆)-Fluoralkoxy, (C₁-C₆)-Mercaptoalkyl,
E Sauerstoff, Schwefel,
Y einen Kohlenwasserstoffrest der Formel
-[CR(3)₂]ₙ-
mit R(3) = H oder (C₁-C₂)-Alkyl und n = 1, 2, 3 oder 4,
X Wasserstoff, F, Cl, (C₁-C₄)-Alkyl,
Z Chlor, Fluor, (C₁-C₄)-Alkyl, (C₁-C₄)-Alkoxy.

5. Verbindung der Formel I nach Anspruch 1, dadurch gekennzeichnet, daß darin bedeuten:
R(1) Wasserstoff, Methyl,
R(2) Methoxy, Methyl,
E Sauerstoff oder Schwefel,
Y einen substituierten oder unsubstituierten Kohlenwasserstoffrest mit zwei bis drei Kohlenstoffatomen der Formel
-[CR(3)₂]ₙ-
mit R(3) = H oder Methyl und n = 2 oder 3,
X Wasserstoff, F, Cl, (C₁-C₃)-Alkyl,
Z Chlor oder Fluor, (C₁-C₃)-Alkyl, (C₁-C₃)-Alkoxy.

6. Verfahren zum Herstellen einer Verbindung I nach Anspruch 1, dadurch gekennzeichnet, daß man
(a) ein aromatisches Sulfonamid der Formel II oder dessen Salz der Formel III 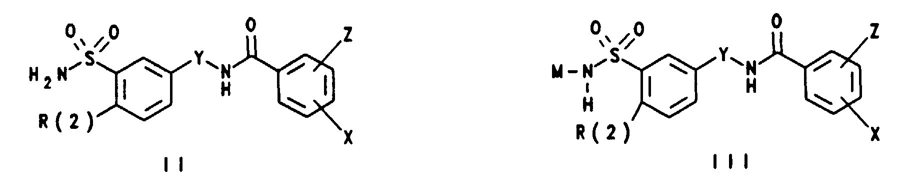 mit einem R(1)-substituierten Isocyanat der Formel IV
R(1) - N = C = O IV
zu einem substituierten Benzolsulfonylharnstoff la umsetzt, oder
(b) einen unsubstituierten Benzolsulfonylharnstoff la (R(1) = H) 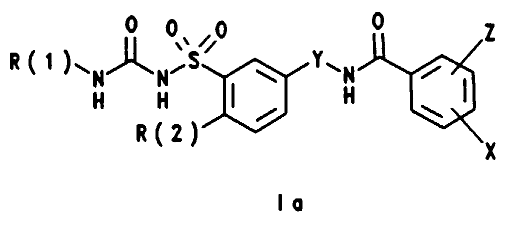 durch Umsetzung eines aromatischen Benzolsulfonamids der Formel II oder dessen Salz III
mit einem Trialkylsilylisocyanat oder Siliciumtetraisocyanat und Hydrolyse des primären siliciumsubstituierten Benzolsulfonylharnstoffs herstellt, oder
(c) einen Benzolsulfonylharnstoff la 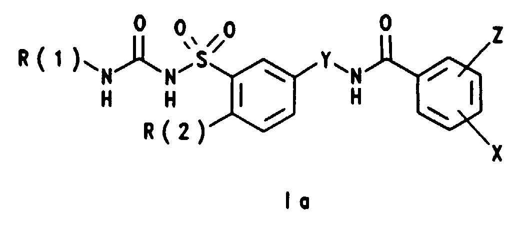 aus einem aromatischen Benzolsulfonamid II oder dessen Salz III aus einem R(1)-substituierten Trichloracetamid der Formel V 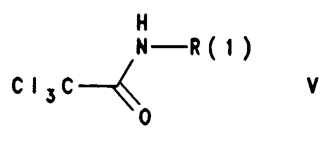 in Gegenwart einer Base darstellt, oder
d) ein aromatisches Sulfonamid der Formel II oder dessen Salz der Formel III 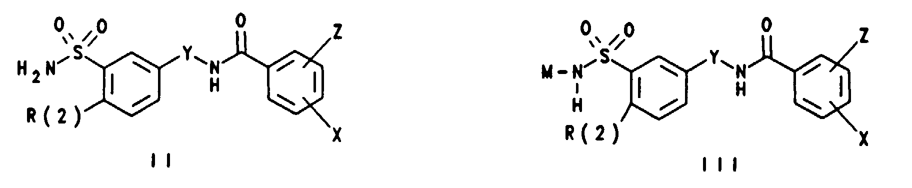
mit einem R(1)-substituierten Thioisocyanat der Formel VI
R(1) - N = C = S VI
zu einem substituierten Benzolsulfonylthioharnstoff 1b umsetzt wobei die Kationen M in den Salzen der formel III Alkali- und Erdalkali-, sowie Tetraalkylammonium ionen sind.

7. Verwendung einer Verbindung I nach Anspruch 1 zur Herstellung eines Medikaments zur Behandlung von Herzrhythmusstörungen.

8. Verwendung einer Verbindung I nach Anspruch 1 zur Herstellung eines Medikaments zur Verhinderung des plötzlichen Herztods.

9. Verwendung einer Verbindung I nach Anspruch 1 zur Herstellung eines Medikaments zur Behandlung von ischämischen Zuständen des Herzens.

10. Verwendung einer Verbindung I nach Anspruch 1 zur Herstellung eines wissenschaftliches Tools zur Inhibition ATP-Sensitiver Kaliumkanäle.

11. Verwendung einer Verbindung I zur Herstellung eines Medikaments zur Behandlung der geschwächten Herzkraft.

12. Verwendung einer Verbindung I zur Herstellung eines Medikaments zur Verbesserung der Herzfunktion nach Herztransplantation.

13. Heilmittel, gekennzeichnet durch eine wirksame Menge an einer Verbindung der Formel I nach Anspruch 1.

## Claims

1. A substituted benzenesulfonylurea or -thiourea of the formula I 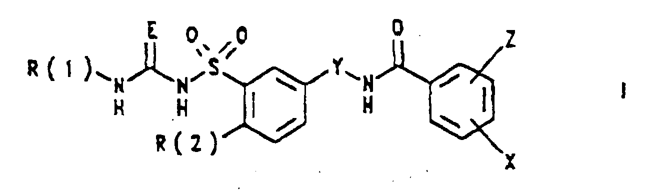 in which
R(1) is hydrogen, methyl, CH₂F, CHF₂ or trifluoromethyl,
R(2) is hydrogen, F, Cl, Br, I, (C₁-C₆)-alkyl, (C₁-C₆)-alkoxy, (C₁-C₆)-mercaptoalkyl, (C₁-C₆)-fluoroalkoxy or (C₁-C₆)-fluoroalkyl,
E is oxygen or sulfur,
Y is a hydrocarbon chain of the formula:
-[CR(3)₂]ₙ-
where R(3) = H or (C₁-C₂)-alkyl and n = 1, 2, 3 or 4,
X is hydrogen, F, Cl, Br, I or (C₁-C₆)-alkyl and
Z is F, Cl, Br, I, NO₂, (C₁-C₄)-alkoxy or (C₁-C₄)-alkyl,
and the physiologically acceptable salts thereof.

2. A compound of the formula I as claimed in claim 1, in which:
R(1) is hydrogen, methyl or trifluoromethyl,
R(2) is hydrogen, (C₁-C₆)-alkyl, (C₁-C₆)-alkoxy, (C₁-C₆)-mercaptoalkyl, (C₁-C₆)-fluoroalkyl, (C₁-C₆)-fluoroalkoxy, F, Cl, Br or I,
E is oxygen or sulfur,
Y is a hydrocarbon chain of the formula
-[CR(3)₂]ₙ-
where R(3) = H or (C₁-C₂)-alkyl and n = 1, 2, 3 or 4,
X is hydrogen, F, Cl or (C₁-C₄)-alkyl and
Z is nitro, F, Cl, (C₁-C₄)-alkyl or (C₁-C₄)-alkoxy.

3. A compound of the formula I as claimed in claim 1, in which:
R(1) is hydrogen or methyl, and
R(2) is hydrogen, fluorine or chlorine,
E is oxygen or sulfur,
Y is a hydrocarbon chain of the formula
-[CR(3)₂]ₙ-
where R(3) = H or (C₁-C₂)-alkyl and n = 1, 2, 3 or 4,
X is hydrogen, F, Cl or (C₁-C₄)-alkyl, and
Z is chlorine, fluorine, (C₁-C₄)-alkyl or (C₁-C₄)-alkoxy.

4. A compound of the formula I as claimed in claim 1, in which:
R(1) is hydrogen or methyl,
R(2) is (C₁-C₆)-fluoroalkyl, (C₁-C₆)-fluoroalkoxy or (C₁-C₆) -mercaptoalkyl,
E is oxygen or sulfur,
Y is a hydrocarbon radical of the formula
-[CR(3)₂]ₙ-
where R(3) = H or (C₁-C₂)-alkyl and n = 1, 2, 3 or 4,
X is hydrogen, F, Cl or (C₁-C₄)-alkyl and
Z is chlorine, fluorine, (C₁-C₄)-alkyl or (C₁-C₄)-alkoxy.

5. A compound of the formula I as claimed in claim 1, in which:
R(1) is hydrogen or methyl,
R(2) is methoxy or methyl,
E is oxygen or sulfur,
Y is a substituted or unsubstituted hydrocarbon radical having two to three carbon atoms of the formula
-[CR(3)₂]ₙ-
where R(3) = H or methyl and n = 2 or 3,
X is hydrogen, F, Cl or (C₁-C₃)-alkyl and
Z is chlorine or fluorine, (C₁-C₃)-alkyl or (C₁-C₃)alkoxy.

6. A process for the preparation of a compound I as claimed in claim 1, which comprises
(a) reacting an aromatic sulfonamide of the formula II or its salt of the formula III 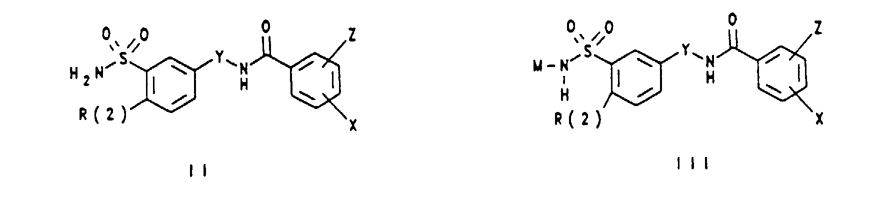 with an R(1)-substituted isocyanate of the formula IV
R(1) - N = C = O IV
to give a substituted benzenesulfonylurea Ia, or
(b) preparing an unsubstituted benzenesulfonylurea Ia (R(1) = H) 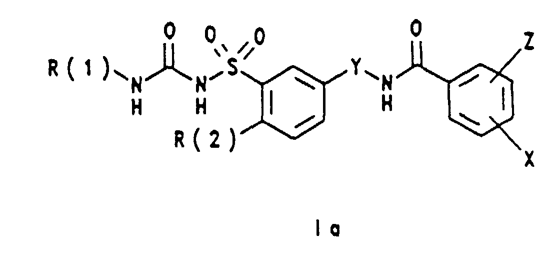 by reaction of an aromatic benzenesulfonamide of the formula II or its salt III
with a trialkylsilyl isocyanate or silicon tetraisocyanate and hydrolysis of the primary silicon-substituted benzenesulfonylurea, or
(c) preparing a benzenesulfonylurea Ia 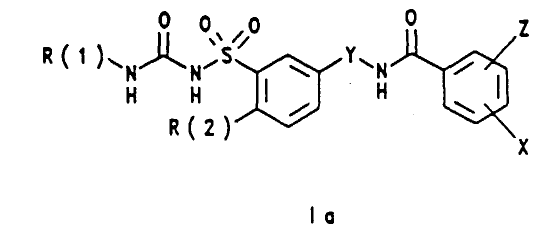 from an aromatic benzenesulfonamide II or its salt III from an R(1)-substituted trichloroacetamide of the formula V 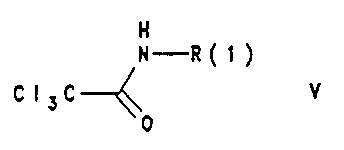 in the presence of a base, or
d) reacting an aromatic sulfonamide of the formula II or its salt of the formula III 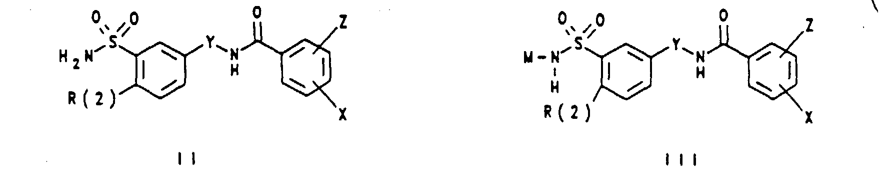 with an R(1)-substituted thioisocyanate of the formula VI
R(1) - N = C = S VI
to give a substituted benzenesulfonylthiourea 1b, the cations M in the salts of the formula III being alkali metal and alkaline earth metal ions, and also tetraalkylammonium ions.

7. The use of a compound I as claimed in claim 1 for the preparation of a medicament for the treatment of disturbances in cardiac rhythm.

8. The use of a compound I as claimed in claim 1 for the preparation of a medicament for prevention of sudden cardiac death.

9. The use of a compound I as claimed in claim 1 for the preparation of a medicament for the treatment of ischemic conditions of the heart.

10. The use of a compound I as claimed in claim 1 for the preparation of a scientific tool for inhibition of ATP-sensitive potassium channels.

11. The use of a compound I for the preparation of a medicament for the treatment of weakened cardiac force.

12. The use of a compound I for the preparation of a medicament for improving cardiac function following a heart transplant.

13. A medicine comprising an active amount of a compound of the formula I as claimed in claim 1.

## Revendications

1. Benzènesulfonylurées et -thio-urées substituées de formule I 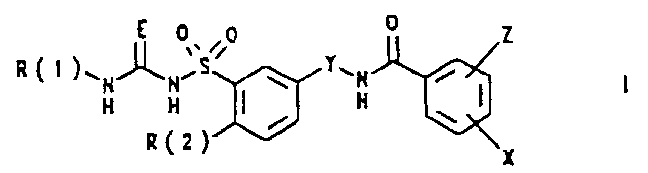 dans laquelle
R(1) représente un atome d'hydrogène ou le groupe méthyle, CH₂F, CHF₂ ou trifluorométhyle,
R(2) représente un atome d'hydrogène, de F, Cl, Br, I ou un groupe alkyle en C₁-C₆, alcoxy en C₁-C₆, mercaptoalkyle en C₁-C₆, fluoroalcoxy en C₁-C₆ ou fluoroalkyle en C₁-C₆,
E représente un atome d'oxygène ou de soufre,
Y représente une chaîne hydrocarbonée de formule:
-[CR(3)₂]ₙ-
dans laquelle R(3) = H ou un groupe alkyle en C₁-C₂ et n = 1, 2, 3 ou 4,
X représente un atome d'hydrogène, de F, Cl, Br, I ou un groupe alkyle en C₁-C₆,
Z représente F, Cl, Br, I, NO₂, un groupe alcoxy en C₁-C₄ ou alkyle en C₁-C₄,
et leurs sels physiologiquement acceptables.

2. Composé de formule I selon la revendication 1, caractérisé en ce que dans cette formule:
R(1) représente un atome d'hydrogène ou le groupe méthyle ou trifluorométhyle,
R(2) représente un atome d'hydrogène, de F, Cl, Br ou I, ou un groupe alkyle en C₁-C₆, alcoxy en C₁-C₆, mercaptoalkyle en C₁-C₆, fluoroalkyle en C₁-C₆ ou fluoroalcoxy en C₁-C₆,
E représente un atome d'oxygène ou de soufre,
Y représente une chaîne hydrocarbonée de formule
-[CR(3)₂]ₙ-
dans laquelle R(3) = H ou un groupe alkyle en C₁-C₂, et n = 1, 2, 3 ou 4,
X représente un atome d'hydrogène ou de F, CI ou un groupe alkyle en C₁-C₄,
Z représente F, CI ou un groupe nitro, alkyle en C₁-C₄ ou alcoxy en C₁-C₄.

3. Composé de formule I selon la revendication 1, caractérisé en ce que dans cette formule:
R(1) représente un atome d'hydrogène ou le groupe méthyle, et
R(2) représente un atome d'hydrogène, de fluor ou de Chlore, E représente un atome d'oxygène ou de soufre,
Y représente une chaîne hydrocarbonée de formule
-[CR(3)₂]ₙ-
dans laquelle
R(3) = H ou un groupe alkyle en C₁-C₂, et n = 1, 2, 3 ou 4,
X représente un atome d'hydrogène, de F ou de Cl, ou un groupe alkyle en C₁-C₄,
Z représente un atome de chlore ou de fluor ou un groupe alkyle en C₁-C₄ ou alcoxy en C₁-C₄.

4. Composé de formule I selon la revendication 1, caractérisé en ce que dans cette formule:
R(1) représente un atome d'hydrogène ou le groupe méthyle,
R(2) représente un groupe fluoroalkyle en C₁-C₆, fluoroalcoxy en C₁-C₆, mercaptoalkyle en C₁-C₆,
E représente un atome d'oxygène ou de soufre,
Y représente un radical hydrocarboné de formule
-[CR(3)₂]ₙ-
dans laquelle R(3) = H ou un groupe alkyle en C₁-C₂, et n = 1, 2, 3 ou 4,
X représente un atome d'hydrogène, de F ou de Cl, ou un groupe alkyle en C₁-C₄,
Z représente un atome de chlore ou de fluor, ou un groupe alkyle en C₁-C₄ ou alcoxy en C₁-C₄.

5. Composé de formule I selon la revendication 1, caractérisé en ce que dans cette formule:
R(1) représente un atome d'hydrogène ou le groupe méthyle,
R(2) représente le groupe méthoxy ou méthyle,
E représente un atome d'oxygène ou de soufre,
Y représente un radical hydrocarboné substitué ou non substitué, ayant 2 ou 3 atomes de carbone, de formule
-[CR(3)₂]ₙ-
dans laquelle R(3) = H ou le groupe méthyle, et n = 2 ou 3,
X représente un atome d'hydrogène, de F ou de Cl, ou un groupe alkyle en C₁-C₃,
Z représente un atome de chlore ou de fluor ou un groupe alkyle en C₁-C₃ ou alcoxy en C₁-C₃.

6. Procédé pour la préparation d'un composé I selon la revendication 1, caractérisé en ce que
(a) on fait réagir un sulfonamide aromatique de formule II ou un sel de celui-ci de formule III 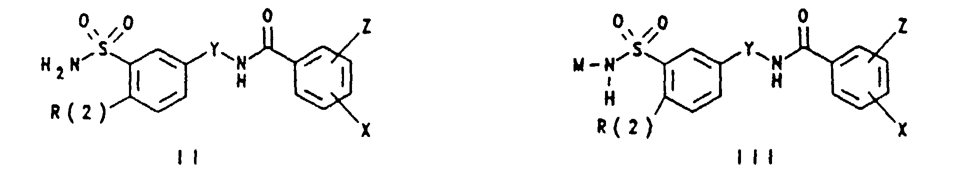 avec un isocyanate substitué par R(1), de formule IV
R(1)-N=C=O IV
pour aboutir à une benzènesulfonylurée substituée la, ou
(b) on prépare une benzènesulfonylurée non substituée la [R(1) = H] 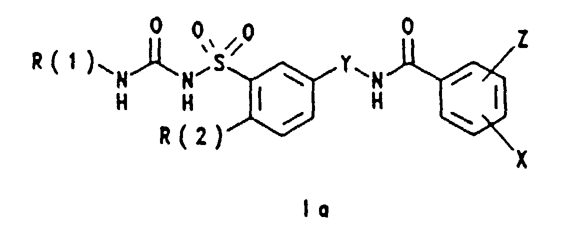 par la réaction d'un benzènesulfonamide aromatique de formule II ou d'un sel III de celui-ci,
avec un trialkylsilylisocyanate ou tétra-isocyanate de silicium, et hydrolyse de la benzènesulfonylurée primaire, substituée par le silicium, ou
(c) on prépare une benzènesulfonylurée la 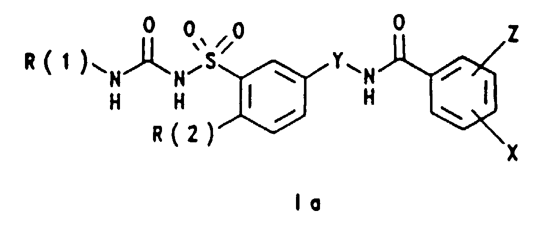 à partir d'un benzènesulfonamide aromatique II ou d'un sel III de celui-ci, et d'un trichloracétamide substitué par le groupe R(1), de formule V 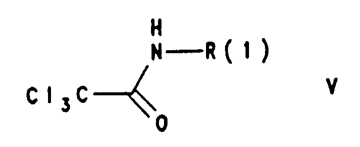 en présence d'une base, ou
(d) on fait réagir un sulfonamide aromatique de formule II ou un sel de formule III de celui-ci 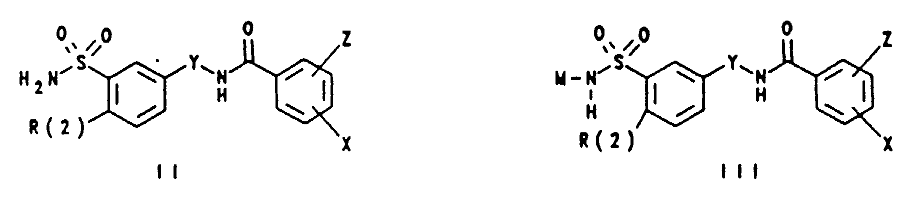 avec un thio-isocyanate substitué par R(1), de formule VI
R(1)-N=C=S VI
pour aboutir à une benzènesulfonylthio-urée substituée Ib (les cations M dans les sels de formule III étant des ions alcalins ou alcalino-terreux, ou tétraalkylammonium).

7. Utilisation d'un composé I selon la revendication 1, pour la fabrication d'un médicament destiné au traitement de troubles du rythme cardiaque.

8. Utilisation d'un composé I selon la revendication 1, pour la fabrication d'un médicament destiné à empêcher la mort subite par arrêt cardiaque.

9. Utilisation d'un composé I selon la revendication 1, pour la fabrication d'un médicament destiné au traitement d'états ischémiques du coeur.

10. Utilisation d'un composé I selon la revendication 1, pour la fabrication d'un outil scientifique pour l'inhibition de canaux potassiques sensibles à l'ATP.

11. Utilisation d'un composé I pour la fabrication d'un médicament destiné au traitement d'une puissance cardiaque affaiblie.

12. Utilisation d'un composé I pour la fabrication d'un médicament destiné à l'amélioration de la fonction cardiaque après une greffe du coeur.

13. Médicament, caractérisé par une quantité efficace d'un composé de formule I selon la revendication 1.
